# EUROPÄISCHE PATENTSCHRIFT

(11) **EP 2 192 921 B1**
(45) Veröffentlichungstag und Bekanntmachung des Hinweises auf die Patenterteilung: **09.11.2016**
(21) Anmeldenummer: 08803690.0
(22) Anmeldetag: 04.09.2008
(51) Int. Cl.: A61K 39/395, A61P 35/00, C07K 16/30, C07K 16/32, C07K 16/28

(54) **INTRAOPERATIVE TRIFUNKTIONALE ANTIKÖRPER-APPLIKATION ZUR PROPHYLAXE INTRAPERITONEALER TUMORZELLDISSEMINATION**
INTRAOPERATIVE TRIFUNCTIONAL ANTIBODY APPLICATION FOR PROPHYLATIC INTRAPERITONAL TUMOUR CELL DISSEMINATION
APPLICATION D'ANTICORPS INTRA-OPÉRATOIRE TRIFONCTIONNELLE POUR LA PROPHYLAXIE DE LA DISSÉMINATION DES CELLULES CANCÉRIGÈNES INTRAPÉRITONÉALES

(30) Priorität: 04.09.2007 EP 07115602
(43) Veröffentlichungstag der Anmeldung: 09.06.2010
(73) Patentinhaber: Lindhofer, Horst, Dr., 80639 München (DE)
(72) Erfinder: LINDHOFER, Horst, 80639 München (DE); HEISS, Markus M., 50968 Köln (DE)
(74) Vertreter: Isarpatent
(86) Internationale Anmeldenummer: PCT/EP2008/061720
(87) Internationale Veröffentlichungsnummer: WO 2009/030734

(56) Entgegenhaltungen:
- WO-A-00/18806
- WO-A-02/20039
- WO-A-2004/014421
- WO-A-2007/093630
- ZEIDLER R ET AL: "THE FC-REGION OF A NEW CLASS OF INTACT BISPECIFIC ANTIBODY MEDIATESACTIVATION OF ACCESSORY CELLS AND NK CELLS AND INDUCES DIRECT PHAGOCYTOSIS OF TUMOUR CELLS" BRITISH JOURNAL OF CANCER, LONDON, GB, Bd. 83, Nr. 2, Juli 2000 (2000-07), Seiten 261-266, XP001053758 ISSN: 0007-0920
- LINDHOFER H ET AL: "BISPECIFIC ANTIBODIES EFFECTIVELY PURGE CANCER CELLS FROM PERIPHERAL BLOOD STEM CELL COLLECTIONS WITHOUT AFFECTING COLONY FORMING UNITS" ANNUAL MEETING INTERNATIONAL SOCIETY FOR EXPERIMENTAL HEMATOLOGY, Bd. 25, Nr. 8, 24. August 1997 (1997-08-24), Seite 879, XP002048523
- OPITZ I ET AL: "Instillation of taurolidine/heparin after laparotomy reduces intraperitoneal tumour growth in a colon cancer rat model" EUROPEAN SURGICAL RESEARCH, Bd. 39, Nr. 3, März 2007 (2007-03), Seiten 129-135, XP009092857 ISSN: 0014-312X

## Beschreibung

Die Erfindung betrifft eine pharmazeutische Zubereitung zur Anwendung in einem Verfahren für die Behandlung der intraoperativen Tumorzelldissemination durch Zerstörung von z. B. durch einen chirurgischen Eingriff intraperitoneal disseminierten Tumorzellen.

Ein bislang ungelöstes Problem in der chirurgischen Onkologie stellt die intraoperative Tumorzelldissemination dar. Es ist akzeptiert, dass es bei den meisten intraabdominellen Tumoroperationen durch den operativen Eingriff zur Ablösung und Dissemination von Tumorzellen kommt, die sich im portalvenösen Blut, im peripheren Blut, im Knochenmark, im Wundblut, aber vor allem innerhalb der Peritonealhöhle verteilen. Tumorbiologisch ist die Dissemination von Zellen in die systemische Blutzirkulation anders zu werten als die Dissemination in die freie Bauchhöhle. Der Nachweis von Tumoreinzelzellen im Blut wurde bereits in den 50er- und 60er Jahren geführt, ohne allerdings einen klaren Bezug zur Prognose darstellen zu können.

Auch mit modernen immunozytochemischen und molekularbiologischen Methoden ist es bislang nicht gelungen, auf überzeugendem Evidenzniveau den Nachweis von systemisch disseminierten Tumoreinzelzellen als Indikatoren einer minimal-residualen Tumorerkrankung mit der tatsächlichen klinischen Prognose des Patienten eindeutig zu korrelieren und dies als Prognose klar zu definieren.

Die Situation ändert sich aber, wenn unterschiedliche Körperkompartimente wie beispielsweise das Knochenmark betrachtet werden. Es gibt Evidenz dafür, dass sich Tumorzellen, die im Knochenmark nachgewiesen worden sind, bereits intestitiell befinden und damit bereits die ersten Schritte in der Extravasation und Invasion von mesenchymaler Matrix erfolgreich absolviert haben. Zumindest für das Mammakarzinom konnte eine Prognoseassoziation zwischen Tumorzellennachweis im Kompartiment Knochenmark und im Auftreten von Skelettmetastasen, aber auch in der Langzeitprognose nachgewiesen werden.

Anders verhält es sich, wenn einzelne Tumorzellen in der freien Bauchhöhle intraperitoneal gefunden werden. Ein zytologischer oder auch immunzytologischer Nachweis von Tumorzellen intraperitoneal bei Tumoren des Gastrointestinaltrakts, wie dem Magenkarzinom und dem kolorektalen Karzinom, sind eindeutige Risikoindikatoren für das Auftreten einer Peritonealkarzinose. Diese intraperitoneale Tumordissemination tritt umso häufiger auf, je ausgeprägter die Infiltrationstiefe des Karzinoms insbesondere in die Serosa ist. Bei makroskopischer Diagnose eines Serosabefalls fanden sich in begleitenden zytologischen Untersuchen mit sehr hoher Wahrscheinlichkeit positive Tumorzellnachweise.

Bei der Tumoroperation kommt nun erschwerend hinzu, dass durch die Präparation der Gewebe und die Manipulation des tumortragenden Organs eine zusätzliche Tumordissemination auftritt. Hier ergibt sich die Fragestellung, wie das Risiko einer solchen Tumordissemination intraoperativ beseitigt werden kann. Bislang waren entsprechende Versuche mit einer intraoperativen Chemoperfusion weniger erfolgreich und verbieten sich auch aufgrund der Komplexität des Verfahrens und des Risikos für eine breite Anwendung. Auch hat sie den Nachteil, nur proliferierende Tumorzellen erreichen zu können.

Aus der WO 02/20039, der WO 00/18806 und der WO 2007/093630 sind trifunktionelle Antikörper bekannt, die zur Zerstörung von Tumorzellen eingesetzt werden. In der WO 02/20039 werden bispezifische und trispezifische Antikörper zur Behandlung von malignem Aszites und Pleuraerguß beschrieben, welche intraperitoneal appliziert werden. Die Antikörper werden allerdings nicht intraoperativ verabreicht, insbesondere nicht intraoperativ direkt in die Bauchhöhle, sondern in Patienten über eine Spritze intraperitoneal appliziert. Die mit einer Operation verbunden Belastungen des menschlichen Körpers (Aktivierung des Immunsystems) treten im Zusammenhang mit dieser Applikation nicht auf.

Die WO 2004/014421 beschreibt die Verwendung eines antikörperenthaltenden Präparates, das gegen ein Tumor-assoziiertes Antigen gerichtet ist, beispielsweise gegen EpCAM, zur intraoperativen Behandlung von Tumorpatienten zur Verhinderung der Disseminierung von Tumorzellen im Rahmen von chirurgischen Eingriffen. Gemäß Anspruch 10 wird dieses Arzneimittel lokal zum Tumorgewebe und/oder zum Wundbereich appliziert.

Zeidler et al., Brit. J. of Cancer, Bd. 83, Nr. 2, Seiten 261 -266 beschreiben die Zerstörung von Tumorzellen durch Aktivierung akzessorischer Zellen und von NK-Zellen mit Hilfe trifunktioneller Antikörper.

Lindhofer et al., Annual Meeting Int. Soc. For Exp. Hematology, Bd. 25, Nr.8, Seite 879 beschreiben trifunktionelle Antikörper zur Entfernung von Tumorzellen aus Blutstammzellsammlungen (PBMSC).

Es ist daher eine Aufgabe der Erfindung, eine pharmazeutische Zusammensetzung bereitzustellen, die zur Anwendung in einem Verfahren zur Behandlung der intraoperativen Tumorzelldissemination durch Zerstörung von Tumorzellen geeignet ist, welche sich entweder vom Tumor, z. B. vom Primärtumor oder seinen Metastasen, ohne Fremdeinwirkung, z. B. durch einen chirurgischen Eingriff, losgelöst, d. h. disseminiert haben und in der Bauchhöhle bzw. im Bauchraum verteilt vorliegen, oder die durch einen chirurgischen Eingriff zur Tumorresektion im Bauchraum bzw. in der Bauchhöhle disseminiert werden, um hierdurch das Rezidivrisiko zu vermindern. Weiterhin soll diese Zusammensetzung zur Prophylaxe von durch einen chirurgischen Eingriff intraperitoneal disseminierter Tumorzellen verursachten Tumorrezidiven geeignet sein.

Diese Aufgabe wird erfindungsgemäß gelöst durch eine pharmazeutische, trifunktionelle bispezifische Antikörper enthaltende Zusammensetzung zur (i) Anwendung in einem Verfahren für die Behandlung der intraoperativen Tumorzelldissemination durch Zerstörung von durch einen chirurgischen Eingriff intraperitoneal disseminierten Tumorzellen und (ii) zur Anwendung in einem Verfahren zur Prophylaxe von durch einen chirurgischen Eingriff intraperitoneal disseminierter Tumorzellen verursachten Tumorrezidiven, wobei der Antikörper intraoperativ direkt lokal in die Bauchhöhle in einer Menge von 1 - 400 µg verabreicht wird und der trifunktionelle, bispezifische Antikörper die nachfolgenden Eigenschaften aufweist:
(a) bindet an eine T-Zelle über CD3;
(b) bindet an zumindest ein tumorassoziiertes Antigen, welches auf intraperitoneal vorkommenden Tumorzellen exprimiert wird;
(c) bindet durch seinen Fc-Teil an Fc-Rezeptor positive Zellen.

Bevorzugte Ausgestaltungen dieser Antikörper werden nachfolgend näher beschrieben.

Diese Zusammensetzung wird erfindungsgemäß intraoperativ appliziert lokal direkt in die Bauchhöhle, d. h. in den Bauchraum. Hierdurch wird eine Zerstörung von z. B. durch den chirurgischen Eingriff bei der Resektion des Primär- und/oder Sekundärtumors disseminierten Tumorzellen erreicht, die ansonsten über die Körperflüssigkeiten in den Körper des Patienten verteilt und die Wahrscheinlichkeit eines erneuten Tumorwachstums, d. h. von Tumormetastasen deutlich erhöhen würden. Die gleiche therapeutische bzw. prophylaktische Anwendung erfolgt zur Zerstörung von Tumorzellen, welche sich vom Primär- oder Sekundärtumor ohne chirurgischen Eingriff losgelöst haben, das heißt die erfindungsgemäß eingesetzten trifunktionellen Antikörper werden intraoperativ direkt lokal in die Bauchhöhle, appliziert, und zwar in Verbindung mit einem chirurgischen Eingriff, welcher der Entfernung des Primär- und/oder Sekundärtumors dient.

Die pharmazeutische, trifunktionelle bispezifische Antikörper enthaltende Zusammensetzung zur erfindungsgemäßen Anwendung in einem Verfahren für die Behandlung der intraoperativen Tumorzelldissemination zur Zerstörung von z. B. durch einen chirurgischen Eingriff intraperitoneal disseminierten Tumorzellen und zur Prophylaxe von Tumorrezidiven, die durch intraperitoneal disseminierte Tumorzellen verursacht werden, unabhängig davon, ob diese durch einen chirurgischen Eingriff verursacht wurden, erfolgt aber immer in Verbindung mit einer Applikation der Antikörper während einer Operation, die zur Entfernung des Primärtumors oder von Tumormetastasen erfolgt.

Diese Applikationsart direkt nach einer Tumorresektion, das heißt intraoperativ , direkt und lokal, hat den großen Vorteil, dass die Zielzellen der trifunktionellen bispezifischen Antikörper, nämlich die frei disseminierten Tumorzellen im Bauchraum, gut erreicht werden können. Es ist bekannt, dass disseminierte Tumorzellen sich bereits innerhalb von 12-24 Stunden an die Peritonialoberfläche ansiedeln und ein erstes primitives Tumorstroma aus Fibrin und interzellulärer Matrix aufbauen. Ein weiterer Vorteil der Anwendung der erfindungsgemäß eingesetzten trifunktionellen bispezifischen Antikörper liegt in der Prophylaxe von z. B. durch den chirurgischen Eingriff intraperitoneal disseminierten Tumorzellen verursachter Tumorrezidive.

Es ist deshalb notwendig, diese Tumorzellen vor diesem Zeitraum von 12-24 Stunden durch die pharmazeutische Zusammensetzung zu erreichen, da durch die Wiederansiedlung der Tumorzellen und den Aufbau der Tumorstroma ihre Erreichbarkeit für die Antikörper schwieriger wird. Die erfindungsgemäß gefundene Lösung, die hier beschriebenen trifunktionellen bispezifischen Antikörper intraoperativ nach Tumorresektion direkt lokal in der Bauchhöhle einzusetzen, bildet den entscheidend neuen Ansatz zu einer erfolgreichen perioperativen Tumortherapie, insbesondere zur Prophylaxe von Tumorrezidiven, insbesondere peritonealen Karzinomatosen, durch Zerstörung frei disseminierter Tumorzellen in der Bauchhöhle.

Bei der peritonealen Karzinomatose handelt es sich um eine Tumorerkrankung, bei der üblicherweise konventionelle Therapieformen wie Chemotherapie, Radiotherapie oder chirurgische Verfahren zu keinen befriedigenden klinischen Ergebnissen führten. Die Prognose für Patienten mit einer peritonealen Karzinomatose ist deshalb auch äußerst ungünstig mit mittleren Überlebensraten von 3-6 Monaten, je nach Art des Primärtumors. Eine der Hauptfaktoren bei der Entwicklung der peritonealen Karzinomatose ist die oben beschriebene intraperitoneale Dissemination von Tumorzellen, die insbesondere durch die chirurgische Behandlung des Tumors, also eine Tumorresektion, verursacht wird, aber auch durch eine Penetration des Tumors oder durch eine seröse Infiltration des Tumors. Persistierende intraperitoneale Tumorzellen, die in das Peritoneum durch Adhäsionsmoleküle und Fibrinschichten eingebettet sind, können sich zu einer makroskopisch sichtbaren Peritonealkarzinomatose entwickeln. Die zurzeit verfügbaren Untersuchungen zeigen, dass die Persistenz intraabdominaler Tumorzellen bei 100% der betroffenen Patienten zu einer Entwicklung einer peritonealen Karzinomatose führen. Dies ist von besonderem Interesse bei Patienten mit einer chirurgischen Resektion des Primärtumors oder eines Tumorrezidivs und führt zu einem erhöhten Risiko für das Auftreten intraabdominaler Tumorzellen. Die einzige Möglichkeit, eine peritoneale Karzinomatose zu verhindern, liegt in der Zerstörung persistierender intraabdominaler Tumorzellen. Erfindungsgemäß wurde überraschend gefunden, dass die hier beschriebenen, an sich bekannten trifunktionellen bispezifischen und trispezifischen Antikörper bei einer intraoperativen Applikation direkt nach Resektion des Tumors disseminierte Tumorzellen effizient zerstören und die Entwicklung einer peritonealen Karzinomatose verhindern können.

Ein weiterer Grund für den erfindungsgemäß vorgeschlagenen Weg einer intraoperativen Verabreichung des trifunktionellen Antikörpers liegt in der postoperativen Bildung intraabdominaler Fibrinplaques und -adhäsionen, welche die disseminierten Tumorzellen bedecken und damit die Wirksamkeit einer Antikörperbehandlung behindern würden. Eine intraoperative Gabe der Antikörper, bevorzugt in Verbindung mit einer frühzeitigen zusätzlichen Verabreichung des trifunktionellen Antikörpers nach der Resektion, ermöglicht eine optimale Verteilung des Antikörpers in der Peritonealhöhle.

Die erfindungsgemäß eingesetzten trifunktionellen bispezifischen Antikörper sind an sich bekannt und werden nachfolgend näher beschrieben. Diese trifunktionellen Antikörper sind auch bereits beschrieben worden, um sie zur Zerstörung von Tumorzellen einzusetzen. Neu und überraschend ist jedoch, dass diese Antikörper auch erfolgreich während eines chirurgischen Eingriffs nach der Tumorresektion in der Bauchhöhle direkt am Operationsort, d. h. lokal in die Bauchhöhle, appliziert werden, um dort intraperitoneal disseminierte Tumorzellen, die beispielsweise durch den Eingriff vom Tumorkörper losgelöst wurden oder bereits dort durch Dissemination vom Tumor vorlagen, zu zerstören und somit ihre Ausbreitung im Körper und das Entstehen von Tumorrezidiven zu verhindern. Erfindungsgemäß werden also die trifunktionellen Antikörper zur Prophylaxe von Tumorrezidiven eingesetzt, die insbesondere aufgrund des chirurgischen Eingriffs und der hierdurch disseminierten Tumorzellen verursacht werden, und zwar während der Operationsphase, solange die Bauchhöhle geöffnet und noch nicht verschlossen ist. Die erfindungsgemäß beschriebene Applikationsart kann aber auch erfolgreich zur Zerstörung von Tumorzellen eingesetzt werden, die sich "selbstständig" ohne Einwirkung von außen vom Tumor losgelöst haben und die sich in der Bauchhöhle befinden. Entscheidend aber ist immer, dass die Applikation während der Operationsphase, also intraoperativ erfolgt, also in einer Phase, während der sich der Patient in einer immunsuppressiven Situation befindet. Die Verabreichung von Antikörpern während der Operation war bisher aus den hier beschriebenen Gründen einer Immunsuppression kontraindiziert.

Gemäß dem Stand der Technik war es nicht üblich, intraoperativ, z. B. direkt lokal in die Bauchhöhle, Antikörper zu applizieren, um Tumorzellen zu zerstören. Der Grund liegt in einer mit dem chirurgischen Eingriff verbundenen immunsuppressiven Situation, die nachfolgend näher beschrieben wird.

Durch die bei einer Operation verursachte lokale Gewebeschädigung wird eine akute Entzündungsreaktion ausgelöst, welche die Aktivierung einer Reihe von Protein-Kaskaden, wie z. B. dem Komplementsystem, der Koagulation, der Fibrinolyse und dem Kinin-Kallikrein-System beinhaltet. Es kommt zur Vasodilatation, gesteigerten Kapillarpermeabilität und zum Verlust von Immunglobolinen, Komplement und anderen Proteinen in das Gewebe. Außerdem wird eine Reihe lokaler Mediatoren freigesetzt, z. B. Cytokine und Eicosanoide, die verschiedene hormonell gesteuerte Prozesse beeinflussen. Man vermutet, dass sich diese Stressantwort bzw. die mit ihr verbundenen Komplikationen gerade bei Hochrisikopatienten wie Tumorpatienten auf die Überlebensrate der Patienten negativ auswirken, in dem sie z. B. das Immunsystem stören. Postoperative Infektionen und ein erhöhtes Metastasenrisiko durch perioperativ verschleppte Tumorzellen, also der intraperitoneal disseminierten Tumorzellen, wirken sich auf die postoperative Morbidität und Mortalität aus.

Die Eröffnung der Bauchhöhle, d. h. die Laparotomie mit nachfolgender Tumorresektion durch übliche operative Techniken, führen zu einer endokrinen Stressantwort und einer perioperativen Immunsuppression. Typisch für Operationen ist eine Leukozytose mit gesteigertem Neutrophilen- und Monocytenanteil und erniedrigtem Eosinophilen- und Lymphozytenanteil. Die Akute-Phase-Proteine sind erhöht, die Komplement-Komponenten erniedrigt und deren Spaltprodukte wiederum erhöht.

Bei der zellvermittelten Immunität erniedrigt sich vor allem nach großen Eingriffen wie einer Laparotomie die absolute Anzahl der Lymphozyten und erreicht erst in einigen Tagen postoperativ erneut ihre Ausgangswerte. Die Anzahl der B- und T-Lymphozyten sinkt, und ihr Verhältnis ändert sich zugunsten der B-Zellen. Eine verringerte T-Zell-Proliferation, begleitet von verschiedenen Lymphokinveränderungen, führt, wie oben beschrieben, zu einem erhöhten Infektionsrisiko, einer erhöhten Mortalitätsrate usw.

In der humoralen Immunität sinkt auch die Anzahl der B-Lymphozyten, ihre Proliferationsantwort ist unterdrückt.

In zahlreichen Studien hat sich eine Depression der zellvermittelten Immunität in der peri- und postoperativen Phase gefunden, verbunden mit einer Reduktion der Zellzahl zirkulierender Lymphozyten und Natürlicher Killerzellen, Beeinträchtigung der Aktivität natürlicher Killerzellen, Depression der T-Zell-Proliferation und verminderter Neutrophilen-Funktion. Es ergibt sich eine signifikante per- und postoperative Lymphopenie, wobei sowohl die B- als auch die T-Lymphozyten betroffen sind.

Die oben dargestellte, beispielsweise von Bettina Gräfe in ihrer Dissertation, 2004 aus dem Universitätsklinikum Münster beschriebene, durch große chirurgische Eingriffe verursachte Immunsuppression müsste eigentlich dazu führen, dass die erfindungsgemäß eingesetzten trifunktionellen bispezifischen und trispezifischen Antikörper ihre Wirksamkeit nicht entfalten können. Diese trifunktionellen Antikörper besitzen nämlich nicht nur die Kapazität zur direkten Zerstörung der Tumorzellen, sondern sind auch in der Lage, eine gegen den zu behandelnden Tumor gerichtete Immunität zu induzieren. Sie sind befähigt, durch ihre Bindung an den Fc-Rezeptor eine Fc-Rezeptor positive Zelle zu aktivieren und hierdurch die Expression von Zytokinen und/oder von costimulatorischen Antigenen zu initiieren bzw. zu erhöhen. Diese Zytokine und costimulatorischen Antigene übertragen an die T-Zelle zumindest ein zweites Aktivierungssignal, das für eine physiologische Aktivierung der T-Zelle benötigt wird, und erhöhen damit die Expression von Aktivierungsmarkern, fördern die Proliferation von T-Zellen und zerstören die Tumorzelle.

Die erfindungsgemäß verabreichten trifunktionellen Antikörper besitzen zwei unterschiedliche Bindungsarme und können daher gleichzeitig zwei verschiedene Antigenstrukturen erkennen und binden. Mit einem Bindungsarm bindet der trifunktionelle Antikörper an einem Tumorantigen auf der Tumorzelle, und mit Hilfe der zweiten Antigenstruktur an T-Lymphozyten und führt beide in einen engen räumlichen Kontext, so dass die T-Zelle in die Lage versetzt wird, die Tumorzelle zu zerstören.

Man konnte davon ausgehen, dass diese hochkomplexe Immunantwort, die zu einer Stimulierung des Immunsystems gegen den Tumor und zu einer direkten Zerstörung der Tumorzelle führt, bei der intraoperativen Verabreichung, bevorzugt bei einer direkten Verabreichung intraoperativ direkt lokal in die Bauchhöhle, nicht wirksam ist, und zwar auf Grund der oben beschriebenen immunsuppressiven Wirkung, in welchen der Körper des Patienten durch die Operation versetzt wird, um die "überschießende" Immunantwort durch den abdominalen chirurgischen Eingriff auszugleichen. Der Körper versucht nämlich, spezifische zelluläre Immunfunktionen, beispielsweise die Aktivität von Phagozyten im Bauchraum, zu reprimieren.

Weiterhin versucht der Körper durch Immunsuppression die erhöhte Bildung von Zytokinen und Akute-Phase-Proteinen zu vermindern, um den Körper wieder in eine normale, physiologische Immunsituation zu bringen. Diese postoperative Suppression des Immunsystems ließ erwarten, dass die Verabreichung der trifunktionellen Antikörper (trAk) nicht zu einer Zerstörung intraperitoneal disseminierter Tumorzellen führt. Aus diesem Grund wurden Antikörper bisher auch intraoperativ lokal nicht angewandt, da ein Erfolg nicht absehbar war. Völlig überraschend zeigte sich jedoch, dass die erfindungsgemäß ausgewählten trifunktionellen bispezifischen Antikörper sehr wohl befähigt sind, trotz der immunsuppressiven Situation ihre immunologischen Funktion wahrzunehmen und eine direkte Zerstörung der Tumorzellen zu bewirken. Diese Funktionen sind insbesondere eine physiologische Aktivierung der T-Zellen, die durch costimulatorische Signale erreicht wird, die von den aktivierten akzessorischen Zellen an die T-Zellen vermittelt werden. Weiterhin werden, neben den T-Zellen, akzessorische Zellen rekrutiert, so dass weitere Killermechanismen wie z. B. eine Phagozytose initiiert werden. Durch diese Mechanismen sind die trifunktionellen Antikörper in der Lage, auch Tumorzellen zu zerstören, welche gegenüber T-Zellen resistent sind. Diese Zerstörung disseminierter Tumorzellen erfolgt trotz der beschriebenen immunsuppressiven Situation nach Tumorresektion in der Bauchhöhle.

Die Erfindung wird unter Bezugnahme auf den Anspruch 1 nachfolgend näher beschrieben. Bevorzugte Ausgestaltungen der Erfindung ergeben sich aus den Unteransprüchen.

Die von der Erfindung umfassten trifunktionellen bispezifischen Antikörper werden während des chirurgischen Eingriffs im Bauchraum eingesetzt. Zunächst erfolgt eine Eröffnung der Bauchhöhle, d. h. eine Laparotomie mit nachfolgender Tumorresektion durch an sich bekannte operative Techniken. Die trifunktionellen bispezifischen Antikörper werden also intraoperativ eingesetzt, d. h. während eines chirurgischen Eingriffs bzw. während einer Operation. Erfindungsgemäß erfolgt die Applikation der Antikörper nach der Resektion des Tumormaterials, jedenfalls vor Verschließung der Bauchhöhle durch direkte Applikation in die Bauchhöhle. Die Ausdrücke "Bauchraum" und "Bauchhöhle" werden hier synonym eingesetzt. Der Kern der Erfindung liegt also in der direkten Kontaktierung des trifunktionellen bispezifischen Antikörpers, wie er in der vorliegenden Anmeldung beschrieben wird, mit der Umgebung der Bauchhöhle und nicht darin, den Antikörper postoperativ zu verabreichen. Lediglich in einer bevorzugten Ausgestaltung der Erfindung wird zusätzlich nach dem chirurgischen Eingriff, d. h. nach Verschluss des Bauchraums, zumindest eine weitere, zweite, dritte usw. Antikörperdosis verabreicht, um eventuell verbliebene, noch nicht zerstörte Tumorzellen zu zerstören.

Bei den Tumoren handelt es sich um Tumoren, die üblicherweise Organe im Bauchraum befallen. Dies sind insbesondere gastrointestinale Tumoren, z. B. Tumoren des Magens, des Dünn- und Dickdarms, der Leber, der Nieren, des Ovars, der Bauchspeicheldrüse, der Appendix und des Pseudoyxoms. Nach Entfernung des Tumors wird der trifunktionelle bispezifische Antikörper in die Bauchhöhle appliziert. Da der Antikörper möglichst mit allen Oberflächen in der Bauchhöhle so vollständig wie möglich in Kontakt treten soll, also nicht nur mit dem Wundbereich und den Tumorgewebe, wird er in einem physiologisch verträglichen Medium, das zum Spülen der Bauchhöhle geeignet ist, appliziert. Bei einem derartigen Medium handelt es sich bevorzugt um eine isotonische bzw. physiologische Lösung, bevorzugt eine isotonische bzw. physiologische Kochsalzlösung. Selbstverständlich sind alle anderen Medien, die sich zur Aufnahme und zur Verteilung des Antikörpers in der Bauchhöhle eignen und die physiologisch verträglich sind, ebenfalls einsetzbar, beispielsweise Pufferlösungen.

Zum Medium können auch weitere pharmazeutisch aktive Stoffe zugegeben werden, beispielsweise Antibiotika, Antimykotika, Anti-Virus-Mittel, antiinflammatorisch wirkende Mittel, analgetisch oder antipyretisch wirkende Mittel, weitere Anti-Tumor-Mittel wie Zytostatika oder andere Antikörper etc. Auch die zusätzliche Gabe von unspezifischen Immunmodulatoren wie GM-CSF, G-CSF usw. ist möglich. Es bleibt dem Fachmann überlassen, über die weiteren pharmazeutisch aktiven Stoffe zu entscheiden, die der Spülflüssigkeit zugegeben werden. Der Fachmann wird über die Art und die Menge dieser weiteren Wirkstoffe in Verbindung mit der zu behandelnden Erkrankung eine Entscheidung treffen. Zusätzlich können pharmazeutisch inaktive Stoffe beigefügt werden, z. B. Stabilisierungsmittel wie Polysorbate, Konservierungsmittel etc.

Das den Antikörper enthaltende Medium wird bei einer direkten lokalen Applikationsform für einen Zeitraum in der Bauchhöhle verbleiben, der ausreichend ist, um einen vollständigen Kontakt zwischen dem Antikörper und den disseminierten Tumorzellen herzustellen. Dieser Zeitraum wird vom behandelnden Arzt bestimmt werden, der seine Entscheidung in Abhängigkeit von der zu behandelnden Erkrankung, den Zustand des Patienten und weiteren Parametern bestimmen wird. Bevorzugt verbleibt das den Antikörper enthaltende Medium für einen Zeitraum von mehreren Minuten, z. B. 1 - 10 Minuten oder 1 - 5 Minuten oder bis zu einer Stunde, möglicherweise auch länger, in der Bauchhöhle. Besonders bevorzugt verbleibt die Flüssigkeit in der Bauchhöhle, wobei sie dann bevorzugt in einer solchen Menge vorliegt, so dass sie vom Körper resorbierbar ist. Üblicherweise werden Flüssigkeiten vom Körper im Bauchraum nach spätestens 24 Stunden weitgehend resorbiert. Bevorzugt wird also die Flüssigkeit in einer solchen Menge eingesetzt, d. h. intraperitoneal, also in die Bauchhöhle, appliziert, dass sie vom Körper resorbierbar ist.

In einer bevorzugten Ausgestaltung der Erfindung wird die Bauchhöhle zumindest einmal mit dem den Antikörper enthaltenden physiologisch verträglichen Medium in Kontakt gebracht, das heißt der Antikörper wird in einem physiologisch verträglichen Medium in die Bauchhöhle appliziert. Das Einbringen erfolgt zum Beispiel durch eine Spritze in den geöffneten Bauchraum (d. h. nicht durch die geschlossene Bauchdecke), durch Eingießen, oder andere, an sich bekannte Applikationsarten. In einer Ausgestaltungsform der Erfindung wird der Antikörper in Form einer Spülflüssigkeit eingesetzt, um durch die Spülung selbst und dem anschließenden Entfernen der Spülflüssigkeit disseminierte Tumorzellen auszutragen. Diese Spülung kann bevorzugt ein oder zweimal wiederholt werden. Bevorzugt verbleibt aber eine für den Patienten akzeptable Menge an Flüssigkeit nach Applikation in der Bauchhöhle, um einen ausreichend langen Kontakt zwischen dem Antikörper und den disseminierten Tumorzellen zu ermöglichen und um alle Oberflächen des Bauchraums möglichst vollständig mit den Antikörpern in Kontakt kommen zu lassen. Wie oben dargestellt wird das physiologisch verträgliche Medium mit dem Antikörper vom Körper resorbiert werden. Alternativ kann es auch aktiv entfernt werden.

Das physiologisch verträgliche Medium kann auf beliebige, vom Arzt bestimmte Weise in die Bauchhöhle appliziert werden. Beispiele hierfür sind das direkte Eingießen des Mediums, eine Applikation über einen Katheter oder über einen permanent eingesetzten Katheter oder über Schlauchsysteme. Weitere bevorzugte Ausgestaltungen sind perkutan eingesetzte Katheter oder subkutan implantierte Portsysteme.

Die Menge des zu applizierenden Antikörpers wird wiederum vom zu behandelnden Arzt in Abhängigkeit von der zu therapierenden Erkrankung bestimmt. Der Antikörper wird in einer Menge von 1-400 µg pro Applikationverabreicht, bevorzugt in einer Menge von 5-100 µg, weiterhin bevorzugt in einer Menge von 10-20 µg. Weitere einsetzbare Mengen sind 10-50 µg, 20-80 µg bzw. 30-60 µg. Die verabreichten Dosismengen des Antikörpers können auch unterschiedlich gewählt werden. Es ist für einen Fachmann selbstverständlich, dass es sich bei diesen Mengenangaben um Richtwerte handelt, die von Patient zu Patient, von Erkrankung zu Erkrankung und von Antikörper zu Antikörper durchaus unterschiedlich sein können. Es gehört zum Fachwissen eines Mediziners, die individuell einzusetzende Antikörpermenge, beispielsweise in Abhängigkeit davon, wie der Patient auf die Antikörperapplikation reagiert, entsprechend auszuwählen.

Die Applikation der Antikörper erfolgt also erfindungsgemäß während der durch den chirurgischen Eingriff verursachten immunsuppresiven Situation und unterscheidet sich damit signifikant von einer im Stand der Technik üblichen Applikationsart, bei der die Antikörper an den Patienten zwar direkt in die Bachhöhle appliziert werden können, aber keine immunsuppressive Umgebung existiert, da die Anwendung nicht intraoperativerfolgt und der Bachraum auch nicht eröffnet ist.

Der Antikörper wird in einer weiteren Ausgestaltungsform der Erfindung postoperativ bevorzugt in zumindest einer weiteren Dosis verabreicht, beispielsweise in Form einer zweiten Dosis zur Aktivierung und Proliferation der Immunzellen und vorzugsweise zumindest in einer dritten Dosis zur Zerstörung verbliebener residueller und disseminierter Tumorzellen. Die Anzahl der weiteren Applikationen hängt zum Beispiel von der Reaktion des Patienten auf die Antikörperapplikation ab, beispielsweise von den Nebenwirkungen, und wird vom zu behandelnden Arzt bestimmt. Bevorzugte Mengen des zu applizierenden Antikörpers werden ebenfalls vom behandelnden Arzt in Abhängigkeit von der zu behandelnden Erkrankung und vom Antikörper bestimmt. Als Richtwerte kann postoperativ als zweite Dosis eine Menge von 1-20 µg, bevorzugt 5-10 µg, verabreicht werden, eine nachfolgende Dosis bevorzugt in einer Menge von 20-100 µg, bevorzugt 30-60 µg, und weitere nachfolgende Verabreichungen bevorzugt in einer Menge von 100-500 µg, bevorzugt 100-300 µg. Die postoperative Applikation kann sowohl intraperitoneal als auch intravenös oder über eine andere an sich bekannte Applikationsart erfolgen.

In einer weiteren Ausgestaltungsform der Erfindung wird der Antikörper postoperativ in einer Menge von 5-1000 µg, bevorzugt 10-500 µg, weiterhin bevorzugt 10-150 µg verabreicht.

Die Verabreichung erfolgt bevorzugt intraperitoneal, wobei beispielsweise eine postoperative Verabreichung 2- bis 5-mal erfolgt.

Die Verabreichung erfolgt in zeitlichen Abständen, wobei z. B. 48-72 Stunden nach der Operation gewählt werden. Beispielsweise wird zwischen den einzelnen Applikationen ein Zeitraum von 2-3 Tagen plusminus mehrere Stunden gewählt. Die Applikationen werden bevorzugt postoperativ über einen Zeitraum von 12-20 Tagen fortgesetzt. Bei Verwendung der erfindungsgemäßen, z. B. murinen Antikörper wird beispielsweise die HAMA (human anti mouse antibody)-Reaktion bestimmt, die im Allgemeinen nach 16-21 Tagen einsetzt, bei einigen Patienten früher, bei anderen später oder überhaupt nicht. Aber selbst eine beginnende HAMA-Reaktion muss die Wirksamkeit während dieses Zeitraums nicht notwendigerweise beeinflussen, da hier u.U. noch keine ausreichenden HAMA-Spiegel erreicht werden. Die bisherigen Erfahrungen zeigen, dass durchaus auch hohe HAMA-Spiegel toleriert werden können, ohne dass relevante Nebenwirkungen oder eine deutliche Abschwächung der Effektivität der erfindungsgemäß eingesetzten trifunktionellen Antikörper beobachtet wurden.

Die erfindungsgemäß eingesetzten trifunktionellen bispezifischen Antikörper sind an sich bekannt. Es handelt sich um Antikörper mit den nachfolgenden Eigenschaften:
(a) bindet an eine T-Zelle über CD3;
(b) bindet an zumindest ein tumorassoziiertes Antigen, welches auf intraperitoneal vorkommenden Tumorzellen exprimiert wird;
(c) bindet durch seinen Fc-Teil an Fc-Rezeptor positive Zellen.

Bei den Antikörpern handelt es sich um sogenannte intakte trifunktionelle bispezifische Antikörper. Durch die Behandlung wird nicht nur die oben beschriebene überraschende direkte Zerstörung der Tumorzellen bei einer intraoperativen Applikation während der immunsuppressiven Phase erreicht, sondern insbesondere durch die nachfolgenden Gaben kann auch eine gegen den zu behandelnden Tumor gerichtete Immunität induziert werden.

Unter "intakte Antikörper" sind solche zu verstehen, die einen funktionellen Fc-Teil besitzen. Bevorzugt handelt es sich hierbei um heterologe Antikörper, d.h. sie sind aus schweren Immunglobulinketten unterschiedlicher Subklassen (-kombinationen, auch Fragmente) und/oder Herkunft (Spezies) zusammengesetzt.

Neben den oben beschriebenen Merkmalen a, b und c weisen die eingesetzten Antikörper in bevorzugten Ausgestaltungen der Erfindung noch die weiteren Merkmale d) und e) auf:
d) aktiviert die Fc-Rezeptor positive Zelle durch seine Bindung an die Fc-Rezeptor positive Zelle, wodurch die Expression von Cytokinen und/oder von kostimulatorischen Antigenen initiiert oder erhöht wird;
e) die kostimulatorischen Antigene und/oder Cytokine übertragen an die T-Zelle zumindest ein 2. Aktivierungssignal, das für eine physiologische Aktivierung der T-Zelle benötigt wird, wobei sich diese Aktivierung in der Hochregulation von Aktivierungsmarkern, der Zerstörung der Tumorzelle und/oder in einer Proliferation der T-Zelle zeigt.

Die erfindungsgemäß verwendbaren Antikörper sind befähigt, die Fc-Rezeptor positive Zelle zu aktivieren, wodurch die Expression von Zytokinen und/oder kostimulatorischen Antigenen initiiert oder erhöht wird.

Die erfindungsgemäß verwendbaren heterologen bispezifischen Antikörper sind an sich bekannt. Sie sind zum Beispiel beschrieben in Lindhofer et al., Blood, 88:4651, 1996 oder Lindhofer et al., J. Immunology, 155:219, 1995.

Auf der Tumorzelle erfolgt eine Hochregulation von MHC 1, sowie eine Aktivierung der intrazellulären Prozessierungsmaschinerie (Proteasom-Komplex) aufgrund der Freisetzung von Zytokinen (wie z.B. INF-γ und TNF-α) in unmittelbarer Nachbarschaft der Tumorzelle. Die Zytokine werden aufgrund trifunktioneller Antikörper-vermittelter Aktivierung von T-Zellen und akzessorischen Zellen freigesetzt. D.h. durch den intakten bsAk werden nicht nur Tumorzellen zerstört oder phagozytiert, sondern indirekt auch deren Tumorimmunogenität erhöht.

Die Aktivierung der Fc-Rezeptor positiven Zelle durch den trAk ist von der Subklasse bzw. der Subklassenkombination des trAk abhängig. Wie in in vitro-Versuchen nachgewiesen werden konnte, sind beispielsweise trAk der Subklassenkombination Maus-IgG2a/Ratte-IgG2b in der Lage, an Fc-Rezeptor positive Zellen zu binden und diese gleichzeitig zu aktivieren, was zur Hochregulation bzw. Neuausbildung (Expression) von kostimulatorischen Antigenen, wie z.B. CD40, CD80 oder CD86, auf der Zelloberfläche dieser Zellen führt (Zeidler et al., J. Immunol., 163:1246, 1999). Dagegen sind bsAk der Subklassenkombination Maus-IgG1/RatteIgG2b zwar in der Lage an Fc-Rezeptor positive Zellen zu binden (Haagen et al., J. Immunology, 1995, 154: 1852-1860), können diese aber offenbar nicht in vergleichbarem Maße aktivieren (Gast et al., Cancer Immunol. Immuntherap., 1995, 40: 390).

Während der intakte trAk die T-Zelle mit einem Bindungsarm (z.B. an CD3 oder CD2) bindet und gleichzeitig aktiviert, können kostimulatorische Signale von der an den Fc-Teil des trAk gebundenen Fc-Rezeptor positiven Zelle an die T-Zelle übermittelt werden. D.h. erst die Kombination von Aktivierung der T-Zelle über einen Bindungsarm des trAk und der gleichzeitigen Übertragung von kostimulatorischen Signalen von der Fc-Rezeptor positiven Zelle auf die T-Zelle, führt zu einer effizienten T-Zellaktivierung. Tumor-spezifische T-Zellen, die an der Tumorzelle ungenügend aktiviert wurden und anergisch sind, können nach der erfindungsgemäßen Behandlung mit intakten bispezifischen Antikörpern oder trispezifischen Antikörpern ebenfalls reaktiviert werden.

Ein weiterer wichtiger Aspekt ist die mögliche Phagozytose, Prozessierung und Präsentation von Tumorbestandteilen durch die vom trAk herangeführten und aktivierten akzessorischen Zellen (Monozyten/Makrophagen, oder dendritische Zellen). Durch diesen klassischen Mechanismus der Präsentation von Antigenen können sowohl tumorspezifische CD4- wie auch CD8-positive Zellen generiert werden. Tumorspezifische CD4-Zellen spielen darüber hinaus eine wichtige Rolle für die Induktion einer humoralen Immunantwort im Zusammenhang mit der T-B-Zell-Kooperation.

Trifunktionale Antikörper können mit einem Bindungsarm an den T-Zellrezeptor-Komplex der T-Zelle, mit dem zweiten Bindungsarm an tumorassoziierte Antigene auf der Tumorzelle binden. Sie aktivieren dabei T-Zellen, die durch Freisetzung von Zytokinen oder Apoptosevermittelnde Mechanismen die Tumorzellen zerstören. Darüber hinaus besteht offenbar die Möglichkeit, dass T-Zellen im Rahmen der Aktivierung mit bispezifischen Antikörpern tumorspezifische Antigene über ihren Rezeptor erkennen und dadurch eine dauerhafte Immunisierung eingeleitet wird. Von besonderer Bedeutung ist dabei der intakte Fc-Teil des trifunktionellen Antikörpers, der die Bindung an akzessorische Zellen wie z.B. Monozyten/Makrophagen und dendritische Zellen vermittelt und diese veranlasst selbst zytotoxisch zu werden und/oder gleichzeitig wichtige kostimulatorische Signale an die T-Zelle weiterzugeben. Auf diese Weise kann offensichtlich eine T-Zellantwort u.U. auch gegen bislang unbekannte, tumorspezifische Peptide induziert werden.

Durch Redirektion von u. U. anergisierten, tumorspezifischen T-Zellen an Tumorzellen mittels trifunktionaler und/oder trispezifischer Antikörper bei gleichzeitiger Kostimulation derartiger T-Zellen durch akzessorische Zellen welche an den Fc-Teil des trifunktionellen oder trispezifische Antikörpers binden, könnte die Anergie von zytotoxischen T-Zellen (CTLs) aufgehoben werden. D.h. eine im Patienten gegen den Tumor existierende T-Zell-Toleranz kann mittels intakter heterologer trifunktioneller Antikörper gebrochen und damit eine dauerhafte Tumorimmunität induziert werden.

Die erfindungsgemäß eingesetzten Antikörper sind bevorzugt zur Reaktivierung von in Anergie befindlichen, tumorspezifischen T-Zellen befähigt. Weiterhin sind sie zur Induktion von tumorreaktiven komplement-bindenden Antikörpern und damit zur Induktion einer humoralen Immunantwort in der Lage.

Die Bindung erfolgt über CD3 an die T-Zelle. Die Fc-Rezeptor positiven Zellen weisen zumindest einen Fcy-Rezeptor Typ I oder III auf.

Erfindungsgemäß einsetzbare Antikörper sind zur Bindung an Monozyten, Makrophagen, dendritische Zellen, "Natural Killer"-Zellen (NK-Zellen) und/oder aktivierte neutrophile Zellen als Fcy-Rezeptor I und/oder III positive Zellen befähigt (Zeidler, 1999, Zeidler 2000 a.a.o.).

Die erfindungsgemäß einsetzbaren Antikörper bewirken, dass die Expression von CD40, CD80, CD86, ICAM-1 und/oder LFA-3 als kostimulatorische Antigene oder/und die Sekretion von Zytokinen durch die Fc-Rezeptor positive Zelle initiiert oder erhöht wird. Die Zytokine sind bevorzugt IL-1, IL-2, IL-4, IL-6, IL-B,IL-12, INF-γ und/oder TNF-α.

Die erfindungsgemäß einsetzbaren Antikörper erkennen und binden an sich bekannte tumor-assoziierte Antigene, die typischerweise auf Tumorzellen im Bauchraum vorkommen. Bevorzugt werden von den Antikörpern tumor-assoziierte Antigene auf einer Tumorzelle erkannt, die der nachfolgenden Gruppe angehören: Her2/neu, CD20, CD30 EpCAM, G250, Proteoglycane, GD2, GD3, MHC II, EGF-R, CA125 und CEA.

Bei dem Antikörper handelt es sich bevorzugt um nachfolgende Antikörper: ein anti-Her2/neu x anti-CD3 Antikörper oder ein anti-EpCAM x anti-CD3-Antikörper ist, der an Fc-γ-Typ I/III-Rezeptoren bindet, jeweils bevorzugt mit der Isotypkombination Ratte-IgG2b/Maus-IgG2a oder Maus-[VH-CH1, VL-CL]-human IgG1-[hinge]- human-IgG3 [Caucasian allotypes G3m(b+g)= no binding to protein A)-[CH2-CH3] /Ratte-[VH-CH1, VL-CL]-human IgG1-[hinge]- human IgG1-[CH2-CH3].

Der trifunktionelle Antikörper ist bevorzugt ein heterologer intakter Ratte/Maus bispezifischer Antikörper.

Mit den erfindungsgemäß einsetzbaren trifunktionellen Antikörpern werden T-Zellen aktiviert und gegen die Tumorzellen redirigiert. Bevorzugt einsetzbare heterologe intakte bispezifische Antikörper werden aus einer oder mehreren der nachfolgenden Isotyp-Kombinationen ausgewählt:
Ratte-IgG2b/Maus-IgG2a,
Ratte-IgG2b/Maus-IgG2b,
Ratte-IgG2b/Maus-IgG3,
Ratte-IgG2b/Human-IgG 1,
Ratte-IgG2b/Human-IgG2,
Ratte-IgG2b/Human-IgG3[orientaler Allotyp G3m(st) = Bindung an Protein A],
Ratte-IgG2b/Human-IgG4,
Ratte-IgG2b/Ratte-IgG2c,
Maus-IgG2a/Human-IgG3[kaukasische Allotypen G3m(b+g) = keine Bindung an Protein A, im folgenden mit * gekennzeichnet]
Maus-IgG2a/Maus-[VH-CH1,VL-CL]-Human-IgG1-[Hinge]-Human-IgG3*-[CH2-CH3]
Maus-IgG2a/Ratte-[VH-CH1,VL-CL]-Human-IgG1-[Hinge]-Human-IgG3*-[CH2-CH3]
Maus-IgG2a/Human-[VH-CH1,VL-CL]-Human-IgG1-[Hinge]-Human-IgG3*-[CH2-CH3]
Maus-[VH-CH1,VL-CL]-Human-IgG1/Ratte-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4[N-terminale Region von CH2]-Human-IgG3*[C-terminale Region von CH2: > AS-Position 251]-Human-IgG3*[CH3]
Ratte-IgG2b/Maus-[VH-CH1,VL-CL]-Human-IgG1-[Hinge-CH2-CH3]
Ratte-IgG2b/Maus-[VH-CH1,VL-CL]-Human-IgG2-[Hinge-CH2-CH3]
Ratte-IgG2b/Maus-[VH-CH1,VL-CL]-Human-IgG3-[Hinge-CH2-CH3, orientaler Allotyp]
Ratte-IgG2b/Maus-[VH-CH1,VL-CL]-Human-IgG4-[Hinge-CH2-CH3]
Human-IgG1/Human-[VH-CH1,VL-CL]-Human-IgG1-[Hinge]-Human-IgG3*-[CH2-CH3]
Human-IgG1/Ratte-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]
Human-IgG1/Maus-[VH-CH1,VL-CL]-Human-IgG1-[Hinge]-Human-IgG4[N-terminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]
Human-IgG1/Maus-[VH-CH1,VL-CL]-Human-IgG1-[Hinge]-Human-IgG2[N-terminale Region von CH2]-Human-IgG3*[C-terminale Region von CH2 : > AS-Position 251]-Human-IgG3*[CH3]
Human-IgG1/Ratte-[VH-CH1,VL-CL]-Human-IgG1-[Hinge]-Human-IgG3*-[CH2-CH3]
Human-IgG1/Maus-[VH-CH1,VL-CL]-Human-IgG1-[Hinge]-Human-IgG3*-[CH2-CH3]
Human-IgG2/Human-[VH-CH1,VL-CL]-Human-IgG2-[Hinge]-Human-IgG3*-[CH2-CH3]
Human-IgG4/Human-[VH-CH1,VL-CL]-Human-IgG4-[Hinge]-Human-IgG3*-[CH2-CH3]
Human-IgG4/Human-[VH-CH1,VL-CL]-Human-IgG4-[Hinge]-Human-IgG4[N-terminale Region von CH2]-Human-IgG3*[C-terminale Region von CH2 : > AS-Position 251]-Human-IgG3*[CH3]
Maus-IgG2b/Ratte-[VH-CH1,VL-CL]-Human-IgG1-[Hinge]-Human-IgG3*-[CH2-CH3]
Maus-IgG2b/Human-[VH-CH1,VL-CL]-Human-IgG1-[Hinge]-Human-IgG3*-[CH2-CH3]
Maus-IgG2b/Maus-[VH-CH1,VL-CL]-Human-IgG1-[Hinge]-Human-IgG3*-[CH2-CH3]
Maus-[VH-CH1,VL-CL]-Human-IgG4/Ratte-[VH-CH1,VL-CL]-Human-IgG4-[Hinge]-Human-IgG4-[CH2]-Human-IgG3*-[CH3]
HumanIgG1/Ratte[VH-CH1,VL-CL]-Human-IgG1[Hinge]-Human-IgG4-[CH2]-HumanIgG3*[CH3]
HumanIgG1/Maus[VH-CH1,VL-CL]-Human-IgG1[Hinge]-Human-IgG4-[CH2]-Human-IgG3*[CH3]
Human-IgG4/Human[VH-CH1,VL-CL]-Human-IgG4-[Hinge]-Human-IgG4-[CH2]-HumanIgG3*-[CH3]

Bei den erfindungsgemäß verwendbaren Antikörpern handelt es sich vorzugsweise um monoklonale, chimäre, rekombinante, synthetische, halbsynthetische oder chemisch modifizierte intakte oder nicht-intakte Antikörper mit beispielsweise Fv-, Fab-, scFv- oder F(ab)₂-Fragmenten.

Bevorzugt werden Antikörper oder Derivate oder Fragmente vom Menschen verwendet oder solche, die derart verändert sind, dass sie sich für die Anwendung beim Menschen eignen (sogenannte "humanized antibodies") (siehe z.B. Shalaby et al., J. Exp. Med. 175 (1992), 217; Mocikat et al., Transplantation 57 (1994), 405).

Die Herstellung der verschiedenen oben genannten Antikörpertypen und -fragmente ist dem Fachmann geläufig. Die Herstellung monoklonaler Antikörper, die ihren Ursprung vorzugsweise in Säugetieren, z.B. Mensch, Ratte, Maus, Kaninchen, Ziege oder Kamelartigen, haben, kann mittels herkömmlicher Methoden erfolgen, wie sie z.B. in Köhler und Milstein (Nature 256 (1975), 495), in Harlow und Lane (Antibodies, A Laboratory Manual (1988), Cold Spring Harbor) oder in Galfre (Meth. Enzymol. 73 (1981), 3) oder der DE 195 31 346 beschrieben sind.

Ferner ist es möglich, die beschriebenen Antikörper mittels rekombinanter DNA-Technologie nach dem Fachmann geläufigen Techniken herzustellen (siehe Kurucz et al., J. Immunol. 154 (1995), 4576; Holliger et al., Proc. Natl. Acad. Sc. USA 90 (1993), 6444).

Die Herstellung von Antikörpern mit zwei verschiedenen Spezifitäten, den sogenannten bispezifischen Antikörpern, ist zum einen durch Anwendung rekombinanter DNA-Technologie möglich, aber auch durch die sogenannte Hybrid-Hybridoma-Fusionstechnik (siehe z.B. Milstein et al., Nature 305 (1983), 537). Hierbei werden Hybridomazellinien, die Antikörper mit jeweils einer der gewünschten Spezifitäten produzieren, fusioniert und Zellklone (Quadrome) identifiziert und isoliert, die Antikörper mit beiden Spezifitäten produzieren.

Das der Erfindung zugrunde liegende Problem kann sowohl durch trifunktionelle bispezifische als auch trispezifische Antikörper gelöst werden, die bevorzugt die gekennzeichneten Eigenschaften und Wirkungen aufweisen. Nachfolgend wird die Herstellung von Antikörpern mit zwei und drei Spezifitäten näher beschrieben. Die Bereitstellung derartiger trifunktioneller Antikörper gehört zum Stand der Technik, und auf die derartige Herstellungstechniken beschreibende Literatur wird hier voll inhaltlich Bezug genommen.

Erfindungsgemäß werden intakte bispezifische Antikörper verwendet. Intakte bispezifische Antikörper sind aus zwei Antikörper-Halbmolekülen (je eine H- und L-Immunglobulinkette), die jeweils eine Spezifität repräsentieren, zusammengesetzt, und besitzen darüber hinaus, wie normale Antikörper auch, einen Fc-Teil mit den bekannten Effektorfunktionen. Sie werden bevorzugt durch die Quadrom-Technologie hergestellt. Dieses Herstellungsverfahren ist beispielhaft in der DE-A-44 19 399 beschrieben. Auf diese Druckschrift, auch bzgl. einer Definition der bispezifischen Antikörper, wird zur vollständigen Offenbarung vollinhaltlich Bezug genommen. Selbstverständlich sind auch andere Herstellungsverfahren einsetzbar, solange sie zu den erfindungsgemäß notwendigen intakten bispezifischen Antikörpern der obigen Definition führen.

Beispielsweise können gemäß dem Herstellungsverfahren von Lindhofer et al., J. Immunology 1995, 155: 219, intakte bispezifische Antikörper in ausreichender Menge produziert werden. Die Kombination von 2 bispezifischen Antikörpern gegen 2 unterschiedliche tumorassoziierte Antigene (z.B. c-erb-B2, und EpCAM) auf den Mammakarzinomzellen minimiert die Gefahr, daß Tumorzellen, die nur ein Antigen exprimieren, unerkannt bleiben.

Weitere Vorteile von intakten bsAk mit der Fähigkeit zur Redirektion von T-Zellen gegenüber den o.g. bsF(ab')2 Fragmenten sind im Einzelnen:
1. An intakte trAk können Fc-Rezeptor positive Zellen binden und einerseits über ADCC (antibody-dependent cell-mediated cytotoxicity) direkt zur Tumorzerstörung beitragen sowie andererseits wie oben näher ausgeführt zur T-Zellaktivierung.
2. Durch intakte T-Zell-redirigierende trAk werden auch anergisierte tumorspezifische T-Zellen an die Tumorzelle herangeführt, die erfindungsgemäß direkt am Tumor reaktiviert werden können. Dies kann durch ein bsF(ab')2-Fragment mit den Spezifitäten anti-CD64Xanti-tumorassoziiertes Antigen nicht erreicht werden.

Die Bindung des trAk an Fcγ-RI besitzt zwei wesentliche Vorteile im Hinblick auf eine optimale anti-Tumorwirksamkeit:
(1) Fcγ-RI positive Zellen besitzen die Fähigkeit, mittels ADCC Tumorzellen zu eliminieren und können insofern synergistisch zur anti-Tumorwirkung der durch den trAk an die Tumorzelle herangeführten cytotoxischen T-Zellen beitragen.
(2) Fcγ-RI positive Zellen (wie z.B. Monozyten/Makrophagen/Dendriten) sind in der Lage, wichtige kostimulatorische Signale, ähnlich wie bei der Antigen-Präsentation, der T-Zelle zu liefern und damit eine Anergisierung der T-Zelle zu verhindern. Es können weiterhin, als erwünschtes Nebenprodukt, aufgrund der durch intakten trAk vermittelten Interaktion von T-Zelle mit akzessorischer Zelle und Tumorzelle sogar T-Zellen, deren T-Zellrezeptor tumorspezifische Peptide (über MHC Antigene auf der Tumorzelle präsentiert) erkennt, stimuliert werden. Die für eine korrekte Aktivierung der T-Zelle notwendigen Kostimuli würden in dieser Konstellation von der akzessorischen Zelle (z.B. Monocyt) geliefert werden. Insofern sollte der erfindungsgemäße eingesetzte Antikörper neben der direkten T-Zellrezeptor-unabhängigen, durch trAk vermittelten Tumorzerstörung ebenfalls tumorspezifische T-Zellen aktivieren und generieren, die nach Abbau der trAk weiterhin im Patienten patrouillieren. D.h. mittels intakter trAk kann ähnlich wie bei gentherapeutischen Ansätzen (z.B. durch Einbau von kostimulatorischen Antigenen wie B-7 in die Tumorzelle) die Tumortoleranz im Patienten durchbrochen werden.

Aus der WO 2004/014421 ist die intraoperative Applikation von Antikörpern zur Verhinderung der Disseminierung von Tumorzellen bekannt. Zunächst ist festzuhalten, dass die Beispiele dieser Anmeldung keinerlei Hinweis auf die Wirksamkeit der behaupteten Therapie geben. Beispiel 1 zeigt die Herstellung eines Lewis-Y Antikörpers. Unter der Überschrift "Anwendung während der Tumorresektion" wird eine intravenöse Behandlung mit dem antikörperenthaltenden Präparat "unmittelbar vor der Operation über einen Zeitraum von 2 Stunden" mit einer Dosis von 50 mg beschrieben. "Danach wird der Tumor innerhalb von 4 Stunden entfernt."

Im Beispiel 2 wird die Hemmung des Tumorwachstums durch Antikörper gegen Lewis-Y in Nacktmäusen beschrieben. In die Nacktmäuse werden Tumorzellen appliziert, und 21 bis 24 Stunden nach Applikation der Tumorzellen erfolgt eine erste intraperitoneale Injektion des antikörperenthaltenden Präparates.

Im Beispiel 4 wird an einem Patienten mit Pleuralerguß intravenös maximal 24 Stunden vor Entfernung des Ergusses das antikörperenthaltende Präparat verabreicht.

In allen drei Beispielen der WO 2004/014421 wird kein Beleg erbracht, dass eine intraoperative Behandlung von Tumorpatienten erfolgreich ist, insbesondere auch die nachfolgend näher beschriebenen zu erwartenden Nebenwirkungen nicht auftreten.

Erfindungsgemäß zu unterscheiden ist auch die erfindungsgemäße intraoperative Applikation dirket lokal in die Bauchhöhle von einer Applikation "direkt in den Wundbereich", wie dies in der WO 2004/014421 beschrieben ist.

Erfindungsgemäß werden, wie vorstehend näher beschrieben, geringe Mengen an trifunktionellen Antikörpern eingesetzt. Im Gegensatz dazu wird im Beispiel 1 der WO 2004/014421 eine Dosis von 50 mg verabreicht, im Beispiel 4 eine Dosis von 100 mg und gemäß Anspruch 9 wird vorgeschlagen, vorzugsweise mindestens 100 mg, meist bevorzugt mindestens 200 mg und insgesamt bis zu 2 g systemisch in einer einmaligen Dosis an den Patienten zu verabreichen.

Ein weiteres wichtiges Unterscheidungskriterium zur WO 2004/014421 liegt in der erfindungsgemäßen Verwendung trifunktioneller T-Zellen aktivierender Antikörper. Wie auf den nachfolgenden Seiten näher beschrieben wird, war mit der Anwendung der erfindungsgemäß eingesetzten trifunktionellen Antikörper ein hohes Risiko verbunden. Die in der WO 2004/014421 eingesetzten Antikörper sind lediglich in der Lage, akzessorische Zellen zu binden und zu aktivieren. Die erfindungsgemäß eingesetzten trifunktionellen Antikörper sind zusätzlich noch in der Lage, T-Zellen zu rekrutieren und zu aktivieren. Da insbesondere T-Zellen ein besonders hohes Gefahrenpotenzial besitzen, können die in der WO 2004/014421 aufgestellten Behauptungen, die wie oben gezeigt noch dazu durch keinerlei experimentelle Daten hinterlegt sind, nicht auf die Antikörper der vorliegenden Erfindung übertragen werden. Ein Beispiel für eine überschießende Immunreaktion bildet der Antikörper TGN1412, bei dessen Verabreichung in einer Phase I-Studie unerwartete und ernsthafte Nebenwirkungen an den Probanden auftraten. In einem Brief an den Herausgeber der Zeitschrift Nature Biotechnology (veröffentlich in Nature Biotechnology, Vol. 24, Seiten 493 bis 496. Nr. 5, Mai 2006) sah sich das Paul-Ehrlich-Institut veranlasst, auf die besondere Gefahr von T-Zell-bindenden Antikörpern hinzuweisen. Insbesondere werden trispezifische Antikörper erwähnt, die ein besonders hohes Gefahrenpotential besitzen.

Zusammenfassend war es für einen Fachmann nicht nahegelegen, an einen Patienten, bei welchem durch die Operation verursacht eine akute Entzündungsreaktion generiert wurde, eine weitere Aktivierung des Immunsystems mit der Verabreichung trifunktioneller Antikörper zu induzieren. Die lebensbedrohlichen Auswirkungen von TGN1412 der Firma Tegenero am 13. März 2006 sprachen gegen die Verwendung bispezifischer trifunktioneller Antikörper, also das Immunsystem über T-Zellen aktivierenden trifunktionellen Antikörpern. Das operative Bauchtrauma führt bereits zu einer inflammatorischen Stimulation von Immunzellen, insbesondere von intraperitoneal vorhandenen Immunzellen am Ort der Traumatisierung. Deshalb ist es nicht nahegelegen und insbesondere aus der WO 2004/014421 nicht ableitbar, dass durch die erfindungsgemäße Applikation trifunktioneller Antikörper keine weitere, kontraindizierte Stimulierung des Immunsystems erfolgt und, obwohl der Körper nachfolgend versucht, die Immunreaktion zu supprimieren, gleichwohl eine positive, durch die trifunktionellen Antikörper hervorgerufene Immunreaktion und eine Zerstörung der Tumorzellen erreichbar ist.

Nachfolgend wird die Erfindung anhand von Ausführungsbeispielen näher erläutert, die bereits an Hand klinischer Daten erfreulicherweise die überraschend positiven Eigenschaften des Einsatzes trifunktioneller bispezifischer Antikörper zeigen.

### 1. Ausführungsbeispiel

Ein 59-jähriger Patient war bei einem ausgeprägten Magenkarzinom operiert worden. Bereits 2 Monate zuvor war das Magenkarzinom mit Peritonealkarzinose diagnostiziert worden, und es war primär eine Chemotherapie (PLF-Schema) durchgeführt worden. Jetzt hatte der Patient zunehmend über eine Magenausgangsstenose geklagt, so dass aufgrund dessen die palliative Magenresektion indiziert worden war. Gleichzeitig war besprochen worden, im Sinne eines Heilversuchs bereits intraoperativ mit der Antikörperapplikation zu beginnen. Nach kompletter Entfernung des Magens mit Kolon transversum und Milz erfolgte dann die intraoperative Installation des Antikörpers. Zur Applikation wurden 100 µg des trifunktionellen Antikörpers Ertumaxomab mit den Merkmalen anti-Her2/neu X anti-CD3 (Isotypkombination MausIgG2a x RatteIgG2b) in 500 ml NaCl-Lösung aufgenommen und intraperitoneal appliziert. Zusätzlich war noch ein intraabdominelles Portsystem implantiert worden, über das dann ab dem 11. postoperativen Tag insgesamt 4 x Antikörper appliziert worden war. Als Indiz für eine Wirksamkeit der Antikörper wurde nach intraoperativer Antikörperapplikation die Wundflüssigkeit/Spülflüssigkeit gesammelt und erneut auf Tumorzellen immunzytochemisch überprüft. Es zeigte sich hier kein Anhalt mehr für Tumorzellen, so dass dies als Wirksamkeitsnachweis dieses Konzeptes betrachtet werden kann.

Der Patient überlebte die Operation trotz dieses extrem fortgeschrittenen Tumorstadiums über 9 Monate, was bei einer mittleren Überlebenszeit von erwarteten 3 Monaten ein sehr positives Ergebnis war. Hinzu kam, dass der Patient über 6 Monate ohne Zeichen für eine wesentliche Passagestörung im Gastrointestinaltrakt lebte.

### 2. Ausführungsbeispiel

Im zweiten Heilversuch wurde eine 35-jährige Patientin mit einem sehr ausgedehnten Magenkarzinom operiert und intraoperativ erneut eine Applikation trifunktionaler Antikörper direkt nach Resektion des Tumors vorgenommen. Bei der Patientin war 4 Tage vor der Operation zur histologischen Sicherung eine diagnostische Laparoskopie durchgeführt worden.

Die intraoperative Lavage direkt nach Laparotomie zeigte allerdings reichlich Tumorzellen, so dass beschlossen wurde, intraoperativ die Antikörperapplikation zu versuchen. Zuvor war nachgewiesen worden, dass die Tumorzellen als Zielantigen Her2/neu exprimieren. Diese ausgiebige Operation umfasste eine Gastrektomie, eine Hemikolektomie, eine Splenektomie und Cholecystektomie. Die intraoperative Antikörperapplikation (10µg Ertumaxomab) wurde von der Patientin problemlos vertragen, so dass sie sich postoperativ erwartungsgemäß erholte. Es wurde beschlossen, am 8. postoperativen Tag die Antikörpertherapie intravenös fortzusetzen. Hierzu wurden 6 µg Ertumaxomab i.v. appliziert, welches allerdings nach 2 Stunden bei einem plötzlichen, raschen Fieberanstieg abgebrochen werden musste.

Trotz Absetzens der Therapie zeigte die Patientin am Folgetag einen erneuten Fieberanstieg, so dass in der weiteren Diagnostik sich als Ursache eine Pneumonie im linken Unterlappen darstellte. Diese wurde dann antibiotisch behandelt. Die Patientin wurde dann nach weiteren 10 Tagen erneut mit 10 µg des Antikörpers Ertumaxomab i.p. behandelt. Der Verlauf war problemlos und wurde von der Patientin gut verkraftet. 2 Tage später wurde eine weitere i.p.-Therapie mit dem gleichen Antikörper auf 100 µg gesteigert. Auch diese Applikation wurde problemlos vertragen, so dass die Patientin am Folgetag entlassen werden konnte.

Die zytologische Untersuchung der Wundflüssigkeit am 3. postoperativen Tag hatte eine vollständige Abreicherung von Tumorzellen gezeigt, welches als Anhalt für die Wirksamkeit der intraoperativen Antikörperapplikation bewertet

### 3. Ausführungsbeispiel

### Versuchsansatz:

Patientin mit Magen-Ca
Enzymatische Isolation von autologen Tumorzellen aus dem Primärtumor
Isolation von PBMC direkt nach Tumorresektion während der OP

### Stimulationsansatz:

50 µg trifunktionale Antikörper / ml Medium
anti-EpCAM X anti-CD3 (Isotypkombination MausIgG2a x RatteIgG2b)
1,0 x 10e6 PBMC / ml
5,0 x 10e4 autologe Tumorzellen in Suspension während Stimulation Stimulation über 12 h

### Messmethode:

Direkter Zytotoxizitäts-Assay (BCECF-Fluoreszenz-Release-Assay)
Inkubationszeit 12h

### Interpretation:

PBMCs wurden während der OP nach Resektion des Magen-Ca entnommen (=Zeitpunkt der operationsbedingten Immunsuppression). Gleichzeitig Herstellung einer autologen Einzelzell-Suspension. Nach Stimulation mit trAk und Targetzellen sind die während der OP entnommenen PBMCs in der Lage, autologe Tumorzellen effektiv zu zerstören. Allogene EpCAM-negative Tumorzellen werden hingegen nicht zerstört. Dieser Effekt funktioniert überraschenderweise direkt nach der Opertion, also in einer immunsuppressiven Situation

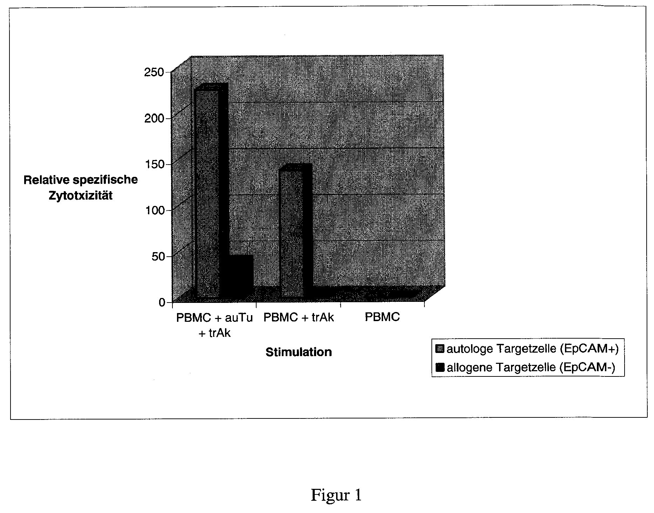

Die beiliegende Figur 1 veranschaulicht diese Wirkungen. Die Figur zeigt:
Stimulationsansätze:
PBMC + trifunktionaler Antikörper + autologe EpCAM+ Targetzellen
PBMC + trifunktionaler. Antikörper
PBMC

### 4. Ausführungsbeispiel

In einer Phase II Studie wurde die adjuvante Behandlung von Patienten während einer chirurgischen Behandlung eines Magenkarzinoms verbunden mit einer intraoperativen Verabreichung des trifunkionellen Antikörpers Catumaxomab verglichen mit einer Behandlung derartiger Patienten ohne intraoperative Verabreichung des Antikörpers.

Insgesamt wurden 55 Magenkarzinompatienten (T2b/T3/T4, N+/-, MO) randomisiert während eines chirurgischen D2-Eingriffs behandelt, entweder ausschließlich mit einem chirurgischen Eingriff oder mit einer intraoperativen Verabreichung des trifunktionalen Antikörpers Catumaxomab. Verabreicht wurden eine intraoperative intraperitoneale Infusion und 4 postoperative interperitoneale Infusionen mit zunehmenden Dosen am Tag 7, 10, 13 und 16. Die primären Ziele waren Sicherheit, Tolerierbarkeit und Machbarkeit.

Catumaxomab ist ein anti-EpCAM x anti-CD3 trifunktioneller monoklonaler Antikörper mit 2 verschiedenen Spezifitäten, die gleichzeitig an das epitheliale Zelladhäsionsmolekül EpCAM auf Tumorzellen und das CD3-Antigen auf T-Zellen binden können. Die Fc-Region besteht aus den Immunglobulin-Isotypen Maus IgG₂ₐ und Ratte IgG_{2b}, die präferentiell FcγI- und III-Rezeptoren auf akzessorischen Zellen, beispielsweise Makrophagen, dendritischen Zellen und NK-Zellen binden können, nicht jedoch an den inhibitorischen FcγIIb-Rezeptor, der auf B-Zellen exprimiert wird.

Von den behandelten 55 Patienten wurden 27 ausschließlich chirurgisch behandelt, 28 mit einer intraoperativen Verabreichung des Antikörpers. Das Vorliegen des Targetantigens

EpCAM wurde in 100% der Patienten bestätigt. 78% (22/28) der mit dem Antikörper behandelten Patienten erhielten alle 5 Infusionen. 40% der treatment-emergent adverse events (TEAEs) trafen direkt nach der intraoperativen Verabreichung auf. TEAEs (CTC-Grad ≥3) traten in 22 Patienten auf (Kontrolle: 10 Patienten). Die häufigsten TEAEs in der Catumaxomab-Gruppe waren Anämie, Pyrexie, SIRS und Abdominalschmerzen. Alle darauf bezogenen ernsthaften TEAEs klangen nach Ende der Behandlung ab, mit Ausnahme einer Nephropathie bei einem Patienten, bei welchem noch geringfügige Spätfolgen verblieben. Es gab keinen Trend zu einer Akkumulation von ernsthaften Nebenwirkungen oder Komplikationen (z.B. bei der Wundheilung) im Vergleich zu denjenigen Patienten, die nur chirurgisch behandelt wurden.

Zusammenfassend kann festgestellt werden, dass die adjuvante intraoperative Behandlung von Magenkarzinomen, bei welchen ein trifunktioneller Antikörper direkt lokal während der Operation in die Bauchhöhle appliziert wird, von den Patienten überraschenderweise ausgezeichnet vertragen wurde. Diese Behandlungsform eröffnet damit eine neue Möglichkeit, das Wiederauftreten intraabdominaler Tumoren nach chirurgischer Entfernung des Magenkarzinoms, verursacht durch Dissemination der Tumorzellen, in Patienten zu vermeiden.

## Patentansprüche

1. Pharmazeutische, trifunktionelle bispezifische Antikörper enthaltende Zusammensetzung zur (i) Anwendung in einem Verfahren für die Behandlung der intraoperativen Tumorzelldissemination durch Zerstörung von durch einen chirurgischen Eingriff intraperitoneal disseminierten Tumorzellen und (ii) zur Anwendung in einem Verfahren zur Prophylaxe von durch einen chirurgischen Eingriff intraperitoneal disseminierter Tumorzellen verursachten Tumorrezidiven, wobei der Antikörper intraoperativ direkt lokal in die Bauchhöhle in einer Menge von 1 - 400 µg verabreicht wird und der trifunktionelle, bispezifische Antikörper die nachfolgenden Eigenschaften aufweist:
(a) bindet an eine T-Zelle über CD3;
(b) bindet an zumindest ein tumorassoziiertes Antigen, welches auf intraperitoneal vorkommenden Tumorzellen exprimiert wird;
(c) bindet durch seinen Fc-Teil an Fc-Rezeptor positive Zellen.

2. Pharmazeutische Zusammensetzung zur Anwendung in einem Verfahren nach Anspruch 1, **dadurch gekennzeichnet, dass** der Antikörper in einem physiologisch verträglichen Medium enthalten ist, das zur Applikation in die Bauchhöhle ausgelegt ist.

3. Pharmazeutische Zusammensetzung zur Anwendung in einem Verfahren nach Anspruch 1 oder 2, **dadurch gekennzeichnet, dass** das den Antikörper enthaltende Medium für einen Zeitraum in der Bauchhöhle verbleibt, der ausreichend ist, um einen Kontakt zwischen dem Antikörper und disseminierten Tumorzellen herzustellen und das Medium mit dem Antikörper anschließend wieder entfernt wird, oder das Medium mit dem Antikörper in der Bauchhöhle verbleibt und dort resorbiert wird.

4. Pharmazeutische Zusammensetzung zur Anwendung in einem Verfahren nach einem oder mehreren der vorhergehenden Ansprüche, **dadurch gekennzeichnet, dass** der Antikörper in einer Menge von 5 - 100 µg, bevorzugt 10 - 50 µg verabreicht wird.

5. Pharmazeutische Zusammensetzung zur Anwendung in einem Verfahren nach einem oder mehreren der vorhergehenden Ansprüche, **dadurch gekennzeichnet, dass** der Antikörper gegen ein Tumorantigen gerichtet ist, ausgewählt aus der Gruppe, bestehend aus Her2/neu, CD20, CD30, EpCAM, G250, Proteoglycane, GD2, GD3, MHC II, EGF-R, CA125 und CEA.

6. Pharmazeutische Zusammensetzung zur Anwendung in einem Verfahren nach einem oder mehreren der vorhergehenden Ansprüche, **dadurch gekennzeichnet, dass** der Antikörper über seinen Fc-Anteil an Fc-γ-Rezeptor Typ I und/oder Typ III positive Zellen bindet.

7. Pharmazeutische Zusammensetzung zur Anwendung in einem Verfahren nach einem oder mehreren der vorhergehenden Ansprüche, **dadurch gekennzeichnet, dass** der bispezifische Antikörper aus einem heterologen bispezifischen Antikörper, bevorzugt einem heterologen Ratte/Maus bispezifischen Antikörper, ausgewählt wird.

8. Pharmazeutische Zusammensetzung zur Anwendung in einem Verfahren nach einem oder mehreren der vorhergehenden Ansprüche, **dadurch gekennzeichnet, dass** ein Antikörper mit einer der nachfolgenden Isotyp-Kombinationen im Fc-Bereich ausgewählt wird:
Ratte-IgG2b/Maus-IgG2a,
Ratte-IgG2b/Maus-IgG2b,
Ratte-IgG2b/human-IgG 1,
Ratte-IgG2b/human-IgG2.

9. Pharmazeutische Zusammensetzung zur Anwendung in einem Verfahren nach einem oder mehreren der vorhergehenden Ansprüche, **dadurch gekennzeichnet, dass** der Antikörper die nachfolgende Isotypkombination besitzt: Mouse-[VH-CH1, VL-CL]-human IgG1-[hinge]-human IgG1-[CH2-CH3]/rat[VH-CH1, VL-CL]-human IgG1-[hinge]-human-IgG3 [Caucasian allotypes G3m(b+g)= no binding to protein A]-[CH2-CH3];
und/oder der Antikörper ein anti-Her2/neu x anti-CD3 Antikörper oder ein anti-EpCAM x anti-CD3-Antikörper ist, der an Fc-γ-Typ I/III-Rezeptoren bindet, jeweils bevorzugt mit der Isotypkombination Ratte-IgG2b/Maus-IgG2a.

10. Pharmazeutische Zusammensetzung zur Anwendung in einem Verfahren nach einem oder mehreren der vorhergehenden Ansprüche, **dadurch gekennzeichnet, dass** der trifunktionelle, bispezifische Antikörper postoperativ in zumindest einer weiteren Dosis verabreicht wird.

11. Pharmazeutische Zusammensetzung zur Anwendung in einem Verfahren nach Anspruch 10, **dadurch gekennzeichnet, dass** der Antikörper postoperativ in Form einer zweiten Dosis zur Aktivierung und Proliferation der Immunzellen und vorzugsweise zumindest einer dritten Dosis zur Zerstörung residueller und disseminierer Tumorzellen verabreicht wird.

12. Pharmazeutische Zusammensetzung zur Anwendung in einem Verfahren nach Anspruch 10 oder 11, **dadurch gekennzeichnet, dass** der Antikörper postoperativ als zweite Dosis in einer Menge von 1-20 µg, bevorzugt 5-10 µg, verabreicht wird, eine nachfolgende Dosis bevorzugt in einer Menge von 20-100 µg, bevorzugt 30-60 µg, und jede weitere nachfolgende Verabreichung bevorzugt in einer Menge von 100-500 µg, bevorzugt 100-300 µg, erfolgt.

13. Pharmazeutische Zusammensetzung zur Anwendung in einem Verfahren nach Anspruch 10, **dadurch gekennzeichnet, dass** der Antikörper postoperativ in einer Menge von 5 - 1000 µg, bevorzugt 10 - 500 µg, weiterhin bevorzugt 10 - 150 µg verabreicht wird, bevorzugt intraperitoneal, und weiterhin bevorzugt die postoperative Verabreichung 2 - 5 mal erfolgt.

## Claims

1. Pharmaceutical composition comprising trifunctional bispecific antibodies for (i) use in a method for treatment of intraoperative tumour cell dissemination by destruction of tumour cells disseminated intraperitoneally by a surgical intervention and (ii) for use in a method for prophylaxis of tumour recurrences caused by tumour cells disseminated intraperitoneally by a surgical intervention, wherein the antibody is administered intraoperatively directly locally into the abdominal cavity in an amount of 1 - 400 µg and the trifunctional, bispecific antibody has the following properties:
(a) binds to a T cell via CD3;
(b) binds to at least one tumour-associated antigen which is expressed on intraperitoneally occurring tumour cells;
(c) binds by its Fc portion to Fc receptor positive cells.

2. Pharmaceutical composition for use in a method according to claim 1, **characterised in that** the antibody is contained in a physiologically acceptable medium which is designed for application into the abdominal cavity.

3. Pharmaceutical composition for use in a method according to claim 1 or 2, **characterised in that** the medium containing the antibody remains in the abdominal cavity for a period of time which is sufficient to establish contact between the antibody and disseminated tumour cells and the medium is subsequently removed again with the antibody, or the medium remains in the abdominal cavity with the antibody and is absorbed there.

4. Pharmaceutical composition for use in a method according to one or more of the preceding claims, **characterised in that** the antibody is administered in an amount of 5 - 100 µg, preferably 10 - 50 µg.

5. Pharmaceutical composition for use in a method according to one or more of the preceding claims, **characterised in that** the antibody is directed against a tumour antigen selected from the group consisting of Her2/neu, CD20, CD30, EpCAM, G250, proteoglycans, GD2, GD3, MHC II, EGF-R, CA125 and CEA.

6. Pharmaceutical composition for use in a method according to one or more of the preceding claims, **characterised in that** the antibody binds via its Fc portion to Fc-γ receptor type I and/or type III positive cells.

7. Pharmaceutical composition for use in a method according to one or more of the preceding claims, **characterised in that** the bispecific antibody is selected from a heterologous bispecific antibody, preferably a heterologous rat/mouse bispecific antibody.

8. Pharmaceutical composition for use in a method according to one or more of the preceding claims, **characterised in that** an antibody having one of the following isotype combinations in the Fc region is selected:
rat IgG2b/mouse IgG2a,
rat IgG2b/mouse IgG2b,
rat IgG2b/human IgG1,
rat IgG2b/human IgG2.

9. Pharmaceutical composition for use in a method according to one or more of the preceding claims, **characterised in that** the antibody has the following isotype combination:
mouse-[VH-CH1, VL-CL]-human IgG1-[hinge]-human IgG1-[CH2-CH3]/rat[VH-CH1, VL-CL]-human IgG1-[hinge]-human IgG3-[Caucasian allotypes G3m(b+g) = no binding to protein A]-[CH2-CH3];
and/or the antibody is an anti-Her2/neu x anti-CD3 antibody or an anti-EpCAM x anti-CD3 antibody which binds to Fc-γ type I/III receptors, in each case preferably with the isotype combination rat IgG2b/mouse IgG2a.

10. Pharmaceutical composition for use in a method according to one or more of the preceding claims, **characterised in that** the trifunctional, bispecific antibody is administered postoperatively in at least one further dose.

11. Pharmaceutical composition for use in a method according to claim 10, **characterised in that** the antibody is administered postoperatively in the form of a second dose for activation and proliferation of the immune cells and preferably at least a third dose for destruction of residual and disseminated tumour cells.

12. Pharmaceutical composition for use in a method according to claim 10 or 11, **characterised in that** the antibody is administered postoperatively as a second dose in an amount of 1 - 20 µg, preferably 5 - 10 ug, a subsequent dose is carried out preferably in an amount of 20 - 100 µg, preferably 30 - 60 µg, and each further subsequent administration is carried out preferably in an amount of 100 - 500 µg, preferably 100 - 300 µg.

13. Pharmaceutical composition for use in a method according to claim 10, **characterised in that** the antibody is administered postoperatively in an amount of 5 - 1,000 µg, preferably 10 - 500 µg, furthermore preferably 10 - 150 µg, preferably intraperitoneally, and further preferably the postoperative administration is carried out 2 - 5 times.

## Revendications

1. Composition pharmaceutique, contenant des anticorps trifonctionnels bispécifiques, destinée (i) à une utilisation dans un procédé de traitement en per-opératoire de la dissémination de cellules tumorales par une destruction des cellules tumorales disséminées intrapéritonéalement par l'intervention chirurgicale et (ii) à une utilisation dans un procédé de prévention de récidives tumorales occasionnées par les cellules tumorales disséminées intrapéritonéalement par l'intervention chirurgicale, dans laquelle l'anticorps est administré directement en per-opératoire localement dans la cavité abdominale en une quantité de 1 à 400 µg et dans laquelle l'anticorps trifonctionnel bispécifique présente les propriétés suivantes :
(a) se lie à une cellule T (CD3) ;
(b) se lie à au moins un antigène associé à la tumeur, lequel est exprimé sur des cellules tumorales présentes intrapéritonéalement ;
(c) se lie par son fragment Fc à des cellules positives au récepteur Fc.

2. Composition pharmaceutique pour une utilisation dans un procédé selon la revendication 1, **caractérisée en ce que** l'anticorps est contenu dans un milieu physiologiquement compatible qui est prévu pour l'application dans la cavité abdominale.

3. Composition pharmaceutique pour une utilisation dans un procédé selon les revendications 1 ou 2, **caractérisée en ce que** le milieu contenant l'anticorps reste dans la cavité abdominale pendant un certain temps, qui est suffisant pour établir un contact entre les anticorps et les cellules tumorales disséminées et que le milieu est ensuite de nouveau enlevé avec l'anticorps, ou le milieu reste avec l'anticorps dans la cavité abdominale et y est résorbé.

4. Composition pharmaceutique pour une utilisation dans un procédé selon l'une ou plusieurs des revendications précédentes, **caractérisée en ce que** l'anticorps est administré en une quantité de 5 à 100 µg, de préférence de 10 à 50 µg.

5. Composition pharmaceutique pour une utilisation dans un procédé selon l'une ou plusieurs des revendications précédentes, **caractérisée en ce que** l'anticorps est dirigé contre un antigène tumoral choisi dans le groupe constitué de Her2/neu, CD20, CD30; EpCAM, G250, protéoglycane, GD2, GD3, MHC II, EGF-R, CA125 et CEA.

6. Composition pharmaceutique pour une utilisation dans un procédé selon l'une ou plusieurs des revendications précédentes, **caractérisée en ce que** l'anticorps se lie à un récepteur Fc-γ de type I et/ou à des cellules positives de type III par l'intermédiaire de son fragment Fc.

7. Composition pharmaceutique pour une utilisation dans un procédé selon l'une ou plusieurs des revendications précédentes, **caractérisée en ce que** l'anticorps bispécifique est choisi parmi un anticorps bispécifique hétérologue, de préférence un anticorps bispécifique hétérologue rat/souris.

8. Composition pharmaceutique pour une utilisation dans un procédé selon l'une ou plusieurs des revendications précédentes, **caractérisée en ce qu'**un anticorps avec les combinaisons isotypiques suivantes dans le fragment Fc est choisi :
Rat-IgG2b/Souris-IgG2a,
Rat-IgG2b/Souris-IgG2b,
Rat-IgG2b/humain-IgG1,
Rat-IgG2b/humain-IgG2.

9. Composition pharmaceutique pour une utilisation dans un procédé selon l'une ou plusieurs des revendications précédentes, **caractérisée en ce que** l'anticorps possède la combinaison isotypique suivante : Souris-[VH-CHI, VL-CL]-humain IgGl-[hinge]-humain IgGl-[CH2-CH3]/rat[VH-CHI, VL-CL]-humain IgGl-[hinge]-humain-IgG3 [allotypes caucasiens G3m(b+g)= no binding to protein A]-[CH2-CH3] ; et/ou l'anticorps est un anticorps anti-Her2/neu x anti-CD3 ou un anticorps anti-EpCAM x anti-CD3, qui se lie à des récepteurs Fc-γ deType I/III, chaque fois, de préférence avec la combinaison isotypique Rat-IgG2b/Souris-IgG2a.

10. Composition pharmaceutique pour une utilisation dans un procédé selon l'une ou plusieurs des revendications précédentes, **caractérisée en ce que** l'anticorps trifonctionnel, bispécifique est administré en postopératoire en au moins une dose supplémentaire.

11. Composition pharmaceutique pour une utilisation dans un procédé selon la revendication 10, **caractérisée en ce que** l'anticorps est administré en postopératoire sous la forme d'une deuxième dose pour l'activation et la prolifération de cellules immunes et de préférence au moins d'une troisième dose pour la destruction de cellules tumorales résiduelles et disséminées.

12. Composition pharmaceutique pour une utilisation dans un procédé selon les revendications 10 ou 11, **caractérisée en ce que** l'anticorps est administré en postopératoire sous forme d'une deuxième dose en une quantité de 1 à 20 µg, de préférence de 5 à 10 µg, une dose suivante en une quantité de préférence de 20 à 100 µg, de préférence de 30 à 60 µg, et que chaque administration suivante est effectuée en une quantité de 100 à 500 µg, de préférence de 100 à 300 µg.

13. Composition pharmaceutique pour une utilisation dans un procédé selon la revendication 10, **caractérisée en ce que** l'anticorps est administré en postopératoire en une quantité de 5 à 1000 µg, de préférence de 10 à 500 µg, plus préférentiellement de 10 à 150 µg, de préférence de manière intrapéritonéale, et plus préférentiellement que l'administration postopératoire s'effectue en 2 à 5 fois.
